**Europäisches Patentamt**

(19) **European Patent Office**

**Office européen des brevets**

(11) Veröffentlichungsnummer: **0 115 549**
**B1**

(12) ## EUROPÄISCHE PATENTSCHRIFT

(45) Veröffentlichungstag der Patentschrift:
25.11.87

(51) Int. Cl.⁴: **A 61 L 17/00,** A 61 L 15/06,
**A 61 K 37/02**

(21) Anmeldenummer: **83101023.6**

(22) Anmeldetag: **03.02.83**

(54) **Paste zur Blutstillung und temporären Defektüberbrückung bei Knochentrauma.**

(43) Veröffentlichungstag der Anmeldung:
**15.08.84 Patentblatt 84/33**

(45) Bekanntmachung des Hinweises auf die Patenterteilung:
**25.11.87 Patentblatt 87/48**

(84) Benannte Vertragsstaaten:
**AT BE CH DE FR GB IT LI LU NL SE**

(56) Entgegenhaltungen:
**DE-B-2 917 037**
**FR-A-2 364 644**
**FR-A-2 374 040**
**FR-A-2 410 477**
**FR-A-2 438 479**
**GB-A-2 014 043**
**US-A-4 268 495**

(73) Patentinhaber: **ETHICON INC., U.S. Route 22,
Somerville New Jersey 08876 (US)**

(72) Erfinder: **Dahlke, Hermann, Dr. med., Kakenhaner
Grund 23, D-2000 Hamburg 65 (DE)**
Erfinder: **Muxfeldt, Hans, Dr. rer.nat., Wilstedter
Weg 28, D-2000 Norderstedt (DE)**

(74) Vertreter: **Vossius & Partner, Siebertstrasse 4
P.O. Box 86 07 67, D-8000 München 86 (DE)**

EP 0 115 549 B1

## Beschreibung

Die Erfindung betrifft eine Paste zur Blutstillung und temporären Defektüberbrückung bei Knochentrauma.

Seit 1892 wird das von Horsley zuerst beschriebene, später verschiedentlich modifizierte Knochenwachs zur mechanischen Blutstillung am Knochen verwendet. Seine ursprüngliche Zusammensetzung war 7 Teile Bienenwachs, 1 Teil Mandelöl und 1 Teil Salicylsäure. Das heute meist benutzte Knochenwachs besteht aus 89 Teilen Bienenwachs und 11 Teilen Isopropylpalmitat. Dieses Präparat löst eine sehr starke Fremdkörperreaktion und chronische Entzündungsreaktionen aus. Innerhalb 12 Wochen wird es nur zu einem geringen Teil resorbiert, während gleichzeitig eine signifikante Verzögerung in der Knochenregeneration eintritt. Vom klinischen Standpunkt aus ist dabei auf das eventuelle Entstehen von Pseudoarthrosen zu achten.

Aus der FR-A-2 374 040 sind Mittel zum Auffüllen (Defektüberbrückung) von Knochentraumen bekannt. Diese Mittel enthalten als Wirkstoff ein gelierbares Polysaccharid und/oder Gelatine.

Auch aus der FR-A-2 364 644 sind Mittel zur Defektüberbrückung von Knochentraumen bekannt, die ein biologisch verträgliches und resorbierbares Polymer (wie es für chirurgisches Nahtmaterial verwendet wird) als Wirkstoff enthält. Das Mittel kann Tricalciumphosphat (vorzugsweise in Mengen von 0,5 bis 5 %, bezogen auf das Polymer) enthalten (vgl. Anspruch 9).

Aus der DE-B-29 17 037 sind Mittel zum Ausfüllen von unter anderem posttraumatischen, osteomyelitischen Höhlen bekannt. Diese Mittel enthalten als Wirkstoff eine nicht resorbierbare polymere Primärkomponente sowie eine resorbierbare Sekundärkomponente und Hydroxylapatit.

Aus der FR-A-2 438 479 sind Mittel zum temporären Ausfüllen von z. B. Knochendefekten bekannt. Diese Mittel enthalten Kollagen sowie ein biologisch resorbierbares Bindemittel für Kollagen; vgl. Anspruch 1. Sie können zusätzlich Calciumphosphat enthalten; vgl. Anspruch 3. Als Bindemittel kommen die auf Seite 6, Zeile 20ff genannten Polymeren in Betracht. Es fehlt jeder Hinweis, daß auch Getreideproteine, z. B. Prolamine, statt der genannten Polymeren verwendet werden können.

Der Erfindung liegt die Aufgabe zugrunde, eine Paste zur Blutstillung und temporären Defektüberbrückung bei Knochentrauma zu schaffen, welche zu einer sicheren Blutstillung am traumatisierten Knochen führt, die Osteogenese nicht hemmt und osteoinduktiv wirkt. Die Lösung dieser Aufgabe beruht auf dem überraschenden Gefund, daß eine Paste, die ein Prolamin sowie feindisperses Tricalciumphosphat oder Hydroxylapatit und ein physiologisch verträgliches Verdünnungsmittel enthält, eine optimale Blutstillung am traumatisierten Knochen bewirkt und hervorragend zur temporären Defektüberbrückung geeignet ist.

Die Erfindung betrifft somit eine Paste zur Blutstillung und temporären Defektivüberbrückung bei Knockentrauma, die dadurch gekennezeichnet ist, daß si 30-60 Gew. -% Prolamin sowie 5-20 Gew. -% Tricalciumphosphat oder Hydroxylapatit in fein disperser Form und ein physiologisch verträgliches Verdünnungsmittel enthält.

Als Verdünnungsmittel wird vorzugsweise ein Gemisch aus einem aliphatischen Alkohol mit 2 bis 5 Kohlenstoffatomen, insbesondere Äthanol, und Wasser in einem Volumenverhältnis von etwa 1 : 1 bis 10 : 0,5 verwendet.

Prolamine bilden die Haupteiweißbestandteile der Getreidekörner bzw. des Getreidemehls. Sie lassen sich - im Gegensatz zu allen anderen Proteinen - aus dem Mehl mit 80 prozentigem Alkohol extrahieren, sind jedoch in wasserfreiem Alkohol oder in Wasser unlöslich. Die wichtigsten Prolamine sind Zein, Gliadin und Hordein. Zein wird erfindungsgemäß bevorzugt. Das Prolamin, vorzugsweise Zein, wird in einer Menge von etwa 3 bis 60, vorzugsweise 5 bis 45 Gewichtsprozent, in der Paste verwendet.

Im Gegensatz zu allen anderen Proteinen sind die Prolamine in verdünnten Alkoholen und anderen Lösungsmitteln löslich, in Wasser aber unlöslich. Ein Gemisch aus Äthanol und Wasser ist das bevorzugte Lösungs- und Verdünnungsmittel, wobei der Wassergehalt zwischen 4 und 50 % liegt, je nachdem, wie schnell ein Ausfällen des Prolamins erwünscht ist. Prolamine ergeben in Äthanol viskose, leicht thixotrope Lösungen. Die Viskosität der Lösungen steigt bei gleichem Prolamingehalt mit der Konzentration des Äthanols. Andererseits steigt bei gegebener Äthanolkonzentration die Viskosität mit der Konzentration des Prolamins.

Die Prolamine sind resorbierbar, so daß eine Pseudoarthrose, wie sie als Folge nicht oder zu langsam resorbierenden Knochenwachses, z. B. beim Sternumverschluß denkbar ist, nicht eintreten kann.

Der Gehalt an feindispersem Hydroxylapatit oder Tricalciumphosphat fördert die Knochenneubildung. Die Korngröße des Hydroxylapatits oder Tricalciumphosphats liegt im allgemeinen in einem Bereich von etwa 3 bis 20 μm, insbesondere von etwa 10 bis 15 μm.

Durch Zugabe von Methylcellulose oder Carboxymethylcellulose in einer Menge von 2 bis 10 Gewichtsprozent läßt sich die Konsistenz der Paste optimieren, so daß ein leicht in dünner Schicht applizierbares Präparat zur Blutstillung am Knochen entsteht. Auch größere Knochendefekte können mit der Paste ausgefüllt und überbrückt werden. Ebenso ist die Reparatur von Trümmerfrakturen damit möglich.

Zur Prophylaxe und Therapie gegen Infektionen kann die Paste mit einem Antibiotikum versetzt werden. Spezielle Beispiele für verwendbare Antibiotika sind Tobramycin, Gentamycin, Cefotaxim und deren Gemische. Das Antibiotikum wird in einer zur vollständigen Hemmung des Wachstums der zu bekämpfenden Keime ausreichenden Menge verwendet.

Der Paste kann anstelle des Antibiotikums oder zusätzlich noch ein Zytostatikum einverleibt werden. Spezielle Beispiele für verwendbare Zytostatika sind Mitomycin, Dichloren, Decarbacin oder Cisplatin. Das Zytostatikum wird in einer zur Hemmung des Tumorwachstums ausreichenden Menge eingesetzt.

Durch den Zusatz feiner Fasern aus einem resorbierbaren Polymer in einer Menge von 1 bis 15 Gewichtsprozent erfolgt eine Konsistenzveränderung derart, daß die Masse form- und knetbar wird und damit auch zur temporären Defektüberbrückung von Knochen dienen kann.

Poly-(lactid-co-glykolid) ist ein hydrolysierbarer, aliphatischer Polyester, der zur Herstellung von resorbierbarem, chirurgischem Nahtmaterial verwendet wird. Andere resorbierbare faserförmige Polymere, die in die erfindungsgemäße Paste eingearbeitet werden können, sind Polyglykolid, Polylactid, Polyoxalat, Polysuccinat, Polydioxanon und Poly-(esteramide).

Die Herstellung der Paste kann auf die in der US-PS 4 268 495 beschriebene Weise erfolgen. Aus dieser Patentschrift sind Prolamin enthaltende injizierbare Lösungen zur Embolisation und Okklusion bekannt.

Die knetbare Paste kann leicht in Knochendefekte gedrückt und dort in die gewünschte Form modelliert werden. Innerhalb eines Zeitraums von etwa 30 bis 90 Minuten erhärtet die Masse.

Die Sterilisation der Paste kann mit Gammastrahlung, Hitze oder durch Zugabe von Propylenoxid in einer Menge von etwa 0,25 bis 0,5 Gewichtsprozent erfolgen.

Die Beispiele erläutern die Erfindung.

**Beispiel 1**

Aus den folgenden Bestandteilen wird eine Paste hergestellt:

| | |
|---|---|
| Äthanol 60 % V/V | 51,7 g |
| Zein | 19,4 g |
| Methylcellulose | 5,0 g |
| Tricalciumphosphat (Korngröße ca. 10 µm) | 15,0 g |
| Propylenoxid | 1,0 g |

In das 60prozentige wäßrige Äthanol wird das Zein unter vorsichtigem Rühren eingetragen. Nach vollständiger Lösung (ca. 12 bis 14 Stunden) wird der pH-Wert mit einer physiologisch verträglichen Säure oder Base auf einen Wert von etwa 6,5 bis 6,8 eingestellt. In die erhaltene viskose Lösung werden nun die Methylcellulose und das Tricalciumphosphat eingetragen. Die erhaltene Paste wird schließlich in der Kälte noch mit 1 g Propylenoxid versetzt und sofort in eine Flasche abgefüllt. Der Paste können noch 1 bis 13 g feiner Fasern aus einem resorbierbaren Polymer, vorzugsweise Polyglykolid oder Polylactid, einverleibt werden.

**Beispiel 2**

Aus den folgenden Bestandteilen wird gemäß Beispiel 1 eine Paste hergestellt:

| | |
|---|---|
| Äthanol 60 % V/V | 51,7 g |
| Zein | 19,4 g |
| Carboxymethylcellulose | 5,0 g |
| Hydroxylapatit (Korngröße ca. 10 µm) | 20,0 g |
| Propylenoxid | 1,0 g |

Die gemäß Beispiel 1 oder 2 hergestellte Paste wird nach einer Längsdurchtrennnug des Brustbeines eines Hundes zur Blutstillung auf den spongiösen Teil des Knochens aufgetragen. Die Paste läßt sich gut auftragen und dringt geschmeidig in die kleinen knöchernen Hohlräume ein. Die diffusen Blutungen aus dem Knochen kommen sofort zum Stillstand.

Bei der Obduktion nach 28 Tagen läßt sich eine partielle Überbrückung des ehemaligen Defekts erkennen. Histologisch kann die blutstillende Substanz nicht mehr nachgewiesen werden.

**Beispiel 3**

Aus den folgenden Bestandteilen wird gemäß Beispiel 1 eine Paste hergestellt:

| | |
|---|---|
| Äthanol 60 % V/V | 51,7 g |
| Zein | 19,4 g |
| Methylcellulose | 5,0 g |
| Tricalciumphosphat (Korngröße ca. 10 µm) | 15,0 g |
| Tobramycin | 0 7 g |
| Propylenoxid | 1,0 g |

Die erfindungsgemäße Paste wird zur Auffüllung einer infizierten Knochenhöhle eingesetzt. Knochenhöhlen im chronisch infizierten Knochenmark sind ein locus minoris resistentiae für rezidivierende Infektionen.

Nach Säuberung des Knochenmarkraumes bis ins Gesunde wird die einsetzende Blutung mit der Paste gestillt. Gleichzeitig wird die Höhle, je nach Größe, entweder prall oder tapetenartig ausgekleidet. Die sofort einsetzende Präzipitation verhindert ein Abfließen der Paste.

Die im Experiment periodisch entnommenen Knochenstücke zeigen im bakteriologischen Versuch eine deutliche Keimwachstumshemmung bis über den 28. Tag hinaus, dann mit nachlassender Tendenz.

**Beispiel 4**

Aus den folgenden Bestandteilen wird gemäß Beispiel 1 eine Paste hergestellt:

| | |
|---|---|
| Äthanol 60 % V/V | 51,7 g |
| Zein | 19,4 g |
| Carboxymethylcellulose | 5,0 g |
| Hydroxylapatit (Korngröße ca. 10 µm) | 20,0 g |
| Mitomycin | 0,8 g |
| Propylenoxid | 1,0 g |

Die erfindungsgemäße Paste wird zur Hemmung von fraglich vorhandenen oder sicher vorhandenen bösartigen Tumoren des Knochens oder Knochenmarks eingesetzt. Im Zusammenhang mit der Einbringung des Zytostatikums Mitomycin kann die Paste entweder in gut fließfähiger Form oder nach Präzipitation bzw. nach Abdünstung des Lösungsmittels in Form von kleinsten festen Partikeln eingebracht werden.

Die fließfähige Form kommt dann in Betracht, wenn die Paste imstande sein soll, eine massive Blutung aus dem Tumorbereich zu unterbinden. Auch hier bietet sich ein Knochentumor an, der entweder bekannt und inoperabel oder operiert worden ist und zur Blutstillung notwendige Maßnahmen erfordert. Dabei ist die prophylaktische Einbringung eines Zytostatikums an die Resektionsgrenze von großem Vorteil, da damit sichergestellt werden kann, daß eine ausreichend lang andauernde Wirksamkeit des Zytostatikums vorhanden ist. Die Reichweite der Wirksamkeit ist mit etwa 1 cm entfernt von der Paste anzusetzen. Da hier eine systemische Wirkung nur bedingt vorhanden ist, kann die Freisetzung des Zytostatikums sehr hoch sein. In der Tat erreicht sie bis zu einem Abstand von etwa 1,5 cm noch mehere Gamma. Dem primär erwünschten Effekt der Blutstillung wird hier ein zweiter, nämlich der der Zytostase, aufgepfropft.

Die feste Pastenform ist dann erwünscht, wenn ein Knochenabschnitt nicht restlos blutfrei gemacht werden darf.

**Patentansprüche**

1. Paste zur Blutstillung und temporären Defektüberbrükkung bei Knochentrauma, dadurch gekennzeichnet, daß sie 3 bis 60 Gewichtsprozent Prolamin sowie 5 bis 20 % Tricalciumphosphat oder Hydroxylapatit in feindisperser Form und ein physiologisch verträgliches Verdünnungsmittel enthält.

2. Paste nach Anspruch 1, dadurch gekennzeichnet, daß das Prolamin Zein und das Verdünnungsmittel ein Gemisch aus einem aliphatischen Alkohol mit 2 bis 5 Kohlenstoffatomen und Wasser ist.

3. Paste nach Anspruch 1 und 2, dadurch gekennzeichnet, daß die Paste 2 bis 10 % Methylcellulose oder Carboxymethylcellulose enthält.

4. Paste nach Anspruch 1 bis 3, dadurch gekennzeichnet, daß die Paste zusätzlich ein Antibiotikum oder ein Zytostatikum enthält.

5. Paste nach einem der Ansprüche 1 bis 4, dadurch gekennzeichnet, daß sie feine Fasern aus einem resorbierbaren Polymer, vorzugsweise Polyglykolid, Polylactid, Poly-(lactid-coglykolid), Polyoxalat, Polysuccinat, Polydioxanon oder Poly-(esteramide), enthält.

6. Verwendung der Paste nach einem der Ansprüche 1 bis 5 zur Herstellung von Präparaten zur Blutstillung und temporären Defektüberbrückung bei Knochentrauma des lebenden menschlichen und tierischen Körpers.

**Claims**

1. A paste for haemostasis and temporary bridging of defects in cases of bone trauma, characterized in that it contains from 3 to 60 % by weight of prolamin and from 5 to 20 % of tricalcium phosphate or hydroxyl apatite in finely dispersed form and a physiologically compatible diluent.

2. The paste as claimed in claim 1, characterized in that the prolamin is zein and the diluent is a mixture of an aliphatic alcohol having from 2 to 5 carbon atoms and water.

3. The paste as claimed in claims 1 and 2, characterized in that the paste contains from 2 to 10% of methyl cellulose or carboxy methyl cellulose.

4. The paste as claimed in claims 1 to 3, characterized in that the paste in addition contains an antibiotic or a cytostatic agent.

5. The paste as claimed in one of claims 1 to 4, characterized in that it contains fine fibers of a resorbable polymer, preferably polyglycolide, polylactide, poly-(lactide-co-glycolide), polyoxalate, polysuccinate, polydioxanone, or poly-(esteramides).

6. The use of the paste as claimed in one of claims 1 to 5 for preparing preparations for haemostasis and temporary bridging of defects in cases of bone trauma of the living human and animal body.

**Revendications**

1. Pâte pour l'hémostase et la palliation temporaire de défauts dans le cas d'un traumatisme osseux, caractérisée en ce qu'elle contient 3 à 60 % en poids de prolamine, ainsi que 5 à 20 % de phosphate tricalcique ou d'hydroxyapatite sous forme finement dispersée et un diluant physiologiquement compatible.

2. Pâte suivant la revendication 1, caractérisée en ce que la prolamine est la zéine et le diluant est un mélange d'un alcool aliphatique de 2 à 5 atomes de carbone avec de l'eau.

3. Pâte suivant les revendications 1 et 2, caractérisée en ce qu'elle contient 2 à 10% de méthylcellulose ou de carboxyméthylcellulose.

4. Pâte suivant les revendications 1 à 3, caractérisée en ce qu'elle contient en outre un antibiotique ou un cytostatique.

5. Pâte suivant l'une quelconque des revendications 1 à 4, caractérisée en ce qu'elle contient des fibres fines faites d'un polymère résorbable, de préférence de polyglycolide, de polylactide, de poly(lactide-co-glycolide), de polyoxalate, de polysuccinate, de polydioxannone ou de poly(esteramide).

6. Utilisation de la pâte suivant l'une quelconque des revendications 1 à 5, pour la confection de préparations pour l'hémostase et la palliation temporaire de défauts dans le cas d'un traumatisme osseux du corps humain ou animal vivant.